# EUROPEAN PATENT APPLICATION

(11) **EP 3 822 358 A1**
(43) Date of publication of application: **19.05.2021**
(21) Application number: 19834155.4
(22) Date of filing: 11.07.2019
(51) Int. Cl.: C12Q 1/02, C12M 3/00, C12N 5/0793

(54) **MEASUREMENT METHOD**

(30) Priority: 12.07.2018 JP 2018132023
(71) Applicant: Tohoku Institute of Technology, Sendai-shi, Miyagi 982-8577 (JP); Stem Cell & Device Laboratory, Inc., Kyoto-shi, Kyoto 600-8491 (JP)
(72) Inventor: SUZUKI, Ikuro, Sendai-shi Miyagi 982-8577 (JP); ODAWARA, Aoi, Sendai-shi Miyagi 982-8577 (JP); AIBA, Kazuhiro, Kyoto-shi Kyoto 600-8491 (JP); TOOI, Norie, Kyoto-shi Kyoto 600-8491 (JP)
(74) Representative: ABG Intellectual Property Law, S.L.
(86) International application number: PCT/JP2019/027477
(87) International publication number: WO 2020/013270

(57) **Abstract**

The present invention provides a method for measuring the propagation of electric activity in neural network and/or axon by using a nerve cell device produced by seeding nerve cells on an oriented fiber sheet. According to the present invention, synapse function can be assessed by using propagation speed of neural network as an index.

## Description

### Technical Field

The present invention relates to a method for measuring propagation of electric activity in a neural network obtained by culturing nerve cells and/or propagation in axon.

### Background Art

Information processing by the neural network plays an important role in the complex function of the brain. Axons which are long projections and short dendritic projections which have complicate branches extend from nerve cells. These neurites are connected to other nerve cells to form a neural network that is a neural circuit. When nerve cells (pre-synaptic cells) fire electrically (generation of action potential), the electrical signals are conducted through the axons and the signals are transmitted at the synapse to the dendritic protrusions of the next nerve cells (post-synaptic cells) from the pre-synaptic cells by the neurotransmitters. The signal transmitted by the neurotransmitters causes the post-synaptic cell to generate action potential again. It is important to measure the state of information processing by a neural network, that is, the state of propagation of electric activity in a neural network for elucidating nerve function and the causes of neurological diseases.

In order to study the nerve function, a neural network formed by culturing nerve cells is used. However, the propagation speed of action potential in neural network is as fast as several m/sec to several hundred m/sec. In order to visualize the propagation of electric activity in such neural network, a method using microelectrode array (abbreviated as MEA hereinafter) having a high time resolution has been reported (non-patent document 1). Since the neurites extend randomly in the neural network formed by culturing nerve cells, the circuit formed has a complicated structure. Therefore, the direction in which the electric activity propagates is very complicated. It is difficult to measure and analyze the direction of propagation. In order to overcome this difficulty, measurement of the propagation of electric activity by a cultured nerve construct in which the direction of extension of the neurites is controlled by using a device subjected to microfabrication for controlling the region where the neurites extend is reported (non-patent documents 2 and 3).

Culturing nerve cells is generally difficult. However, various means for promoting the proliferation of nerve cells have been reported by providing a scaffold to which nerve cells adhere. For example, it has been reported that the proliferation of nerve cells is promoted by seeding nerve cells in a scaffold in which microfibers comprising polycaprolactone or polycaprolactone mixed with gelatin are aligned in porous three-dimensional hydrogel (non-patent document 4). In addition, there are several reports relating to scaffold materials used as scaffolds for cells for application to medical materials and the like. For example, cell culture or tissue regeneration is effectively performed by using a cell scaffold material composed of nanofibers which is composed of polyolefin, polyamide, polyurethane, polyester, fluorine-based polymer, polylactic acid, polyvinyl alcohol, or the like, or the nanofiber to which the protein component is adsorbed (patent document 1). A three-dimensional cell culture using a cell scaffold material having a hollow fiber membrane mesh and a nanofiber layer enables the supply of nutrients and oxygen to the cultured cells and the removal of metabolic waste from the cultured cells with high efficiency (patent document 2). A large amount of pluripotent stem cells are supplied and cell death is suppressed by using a cell scaffold material composed of a nanofiber containing gelatin, collagen or cellulose, or the crosslinked nanofiber (patent document 3). A cell scaffold material coated with nanofibers composed of polyglycolic acid (used as support) or gelatin etc. is used to improve the proliferation rate of human pluripotent stem cells (patent document 4).

### Prior Art Literatures

### Patent Documents

1: Japanese patent publication No. 2006-254722
2: Japanese patent publication No. 2011-239756
3: Japanese patent publication No. 2013-247943
4: WO2016/068266

### Non-patent Documents

1: Nat Commun. 2013; 4: 2181. doi: 10.1038/ncomms3181.
2: RSC Advances. Jan 2013, Vol. 3, No. 45: 23620.
3: Integr Biol (Camb). 2015 Jan; 7 (1): 64-72. doi: 10.1039/c4ib00223g.
4: Lee, S-. J., et al. Tissue Eng Part A. 2017 Jun; 23(11-12): 491-502.

### Summary of the Invention

### Problems to be Solved by the Invention

In the case of measuring by using microelectrode array (MEA) the propagation of electric activity in a neural network formed by culturing nerve cells and/or the propagation in axon, there is a method of preparing a culture nerve construct in advance and culturing while securing a region such as minute passage where neurites extend (non-patent document 3). In this method, the biological tissue structure is not reflected, since the culture region for nerve cell body and the culture region for the extending neurites are separated. Further, the procedures of seeding the nerve cells on the electrode array and preparing a culture nerve construct are complicated, so that cell death may have occurred depending on the kind of nerve cells. In addition, since the flow of the culture medium into the minute passage in which neurites extend is not sufficient, the oxygen and nutrition do not sufficiently reach the inside of the passage, so that the cell culture in inhibited. As a result, the culture region is large and it is difficult to form a large scale of neural network in which a large number of synapses exist. On the other hand, in a small scale of neural network having a narrow culture region, an MEA having a high spatial resolution such as a CMOS chip is required because the range in which propagation of electric activity can be observed is limited. Moreover, in optical measurement using a membrane potential-sensitive dye capable of measuring neural activity, it is considered that the time resolution is insufficient in a small scale of neural network. The purpose of the present invention is to overcome these problems.

### Means for Solving the Problems

As a result of intensive studies, the inventors have found out that the above problems can be overcome by using a nerve cell device obtained by culturing nerve cells on a fiber sheet as a cell scaffold in measuring the propagation of electric activity in a neural network and/or an axon. Thus, the present invention is completed.

That is, the purpose of the present invention is achieved by the following inventions.
(1) A method for measuring propagation of electric activity in a neural network and/or an axon using a nerve cell device which comprises a cell scaffold and nerve cells.
(2) The method according to (1), wherein the cell scaffold is a fiber sheet formed of a polymeric material.
(3) The method according to (2), wherein the fiber sheet has an orientational structure, a non-orientational structure, or a mixed structure of orientational and non-orientational structure.
(4) The method according to (2) or (3), wherein the fiber sheet is coated with an extracellular matrix protein selected from polylysine, polyornithine, laminin, fibronectin, Matrigel® and Geltrex®.
(5) The method according to (2) or (3), wherein the fiber sheet is coated with polyethyleneimine.
(6) The method according to any one of (1) to (5), wherein the nerve cells formed a three-dimensional structure on a cell scaffold and/or in a cell scaffold.
(7) The method according to any one of (1) to (6), wherein the nerve cells are the nerve cells derived from primary cultured cells or pluripotent stem cells.
(8) The method according to (7), wherein the nerve cells derived from primary cultured cells or pluripotent stem cells are those derived from mammals.
(9) The method according to any one of (1) to (8), wherein the nerve cells comprise glutamatergic, dopaminergic, γ-aminobutyric acidergic, monoaminergic, histaminergic or cholinergic nerve cells.
(10) The method according to any one of (1) to (9), wherein the nerve cell device further comprises a frame that holds the periphery of the device.
(11) The method according to (10), wherein the frame has a size of 2 mm×2 mm to 15 mm×15 mm in terms of length × width, and has circular or polygonal shape.
(12) The method according to any one of (1) to (11), wherein the nerve cell device is in contact with a plurality of regularly arranged electrodes.
(13) The method according to (12), wherein the regularly arranged electrodes are microelectrode arrays.
(14) A method for assessing neural activity using the method according to any one of (1) to (13).

### Effects of the Invention

The nerve cell device used in the present invention can extend the neurites along the fiber constituting a fiber sheet as a cell scaffold. Thus, oxygen and nutrients are efficiently supplied to the cells through the fibers to stably form a large scale of neural network. Moreover, since there is no need to separate a culture region for the nerve cell body from a region where the neurites such as minute passages extend, a neural network is formed in a state close to biological tissue. It is also possible to form a neural network in which myelin is formed around the axons, which is similar to the biological tissue. Therefore, the method for measuring the propagation of action potential according to the present invention can be widely applied to the central and peripheral nerve cells.

According to the present invention, since a previously produced nerve cell device is mounted on MEA at the time of measurement, the action potential can be measured easily and efficiently. In the present invention, a large scale of neural networks is used, so that neural network propagation can be captured in a wide range. Therefore, the propagation delay time as much as several hundred m/sec can be detected. Their application to optical measurement using membrane potential-sensitive dye is also possible.

Accordingly, a method for performing easily and stably the analysis of propagation of electric activity in a neural network and propagation in axon is provided by the present invention. As a result, a method for assessing synapse function by using the propagation speed as an index of electric activity in a neural network is provided. According to the method of the present invention, assessment of functional disorders in axon and synapse and drug assessment on these functional disorders can be performed effectively.

### Brief Description of the Drawings

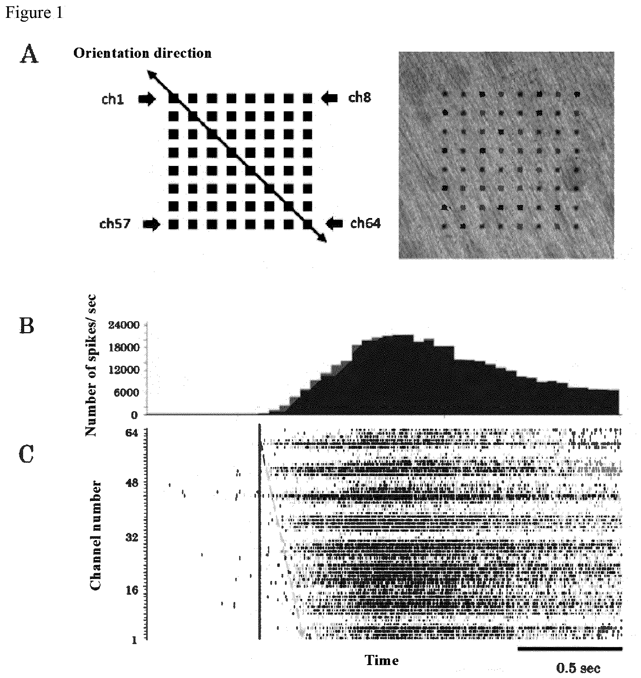

FIG. 1 is a diagram showing the results obtained by measuring the propagation of action potential in a neural network with MEA. Nerve cells derived from human iPS cells were seeded on an oriented PLGA fiber sheet at a density of 8×10⁵ cells/cm² and cultured for 6 weeks to produce a nerve cell device.
(A) is a diagram showing the arrangement of microelectrodes in MEA and the orientation direction of fibers constituting the PLGA fiber sheet. The right figure is an optical microscope photograph (magnification: ×4) showing a state in which a fiber sheet culturing nerve cells is placed on MEA. 64 (8×8) microelectrodes are arranged on the flat surface of the MEA. The left figure shows the arrangement of the microelectrodes. The channel numbers are successively increased from the left end to the right end, and from the upper stage to the lower stage. For example, channels 1 to 8 (ch1-ch8) are arranged in the direction from the left end to the right end of the uppermost stage, and channels 57 to 64 (ch57-ch64) are arranged in the direction from the left end to the right end of the lowermost stage. The orientation direction of the fibers is indicated by two arrows.
(B) is a diagram showing a time change of integrated values of the number of spikes measured by the 64 microelectrodes of the MEA (vertical axis: array-wide spike detection rate (AWSDR, number of spikes/sec).
(C) is a diagram showing a spike measured over time with each microelectrode of the MEA. The vertical dotted line indicates the start point of propagation of action potential in the neural network.

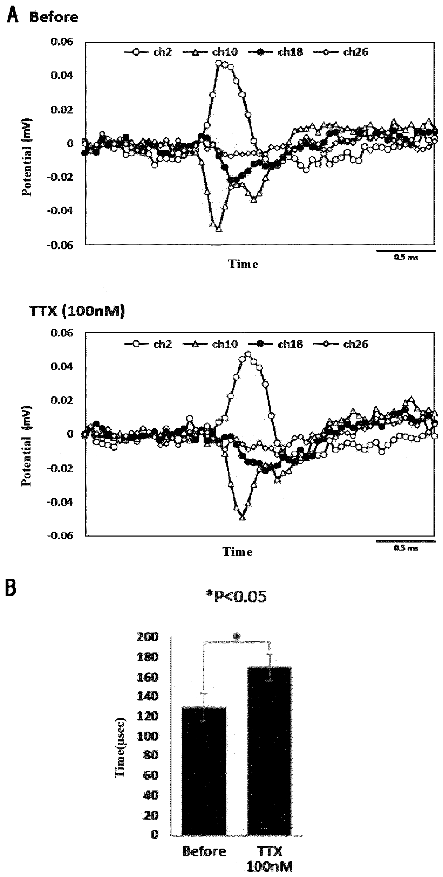

FIG. 2 is a diagram showing the results of the effect of tetrodotoxin (TTX) on the propagation speed of action potential in axon measured by MEA (channel numbers 2, 10, 18 and 26). Nerve cells derived from human iPS cells were seeded on an oriented PLGA fiber sheet at a density of 8×10⁵ cells/cm² and cultured for 6 weeks to produce a nerve cell device.
(A) shows the results obtained by measuring the change in intracellular potential over time before the addition of TTX (Before) and after the addition of TTX (100 nM).
(B) shows the propagation speed of action potential in axon before the addition of TTX (Before) (n=12) and after the addition of TTX (100 nM) (n=9). Each result is indicated by an average value ± standard error.

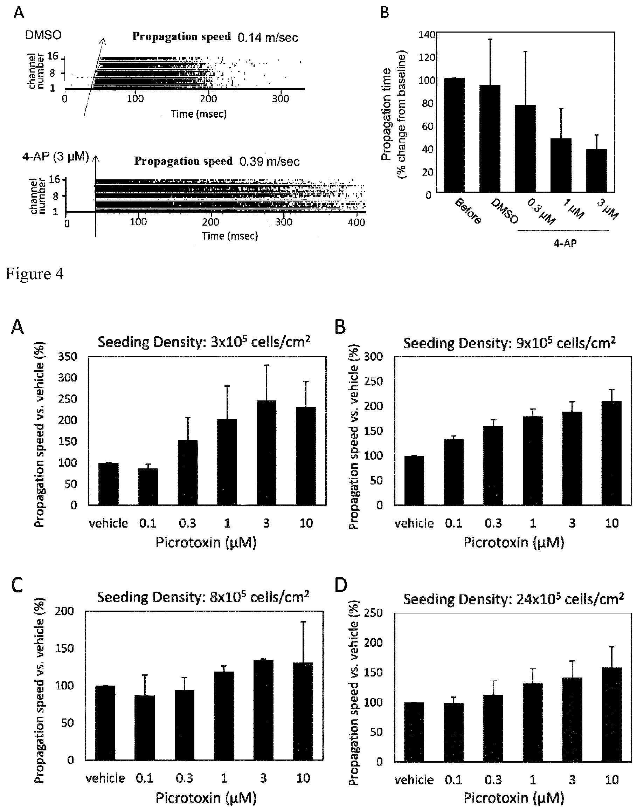

FIG. 3 is a diagram showing the results of measuring by MEA (16 channels) the effect of 4-aminopyridines (4-AP) on the propagation speed of action potential in a neural network included in a nerve cell device.
(A) is a raster plot diagram of the electrical signal measured by MEA after the addition of DMSO (upper figure) or 4-AP (lower figure).
(B) is a diagram showing the results of measuring the time taken by the action potential in a neural network from the channel number 1 of the MEA to the channel number 16 (the channel at the diagonal of the MEA) after the addition of 4-AP (0.3, 1, and 3 µM) or DMSO and before the addition of the drug. The results are relative values in which the action potential arrival time in case of before the addition of the drug is taken as 100, and indicated by the average ± standard error (n=4). The decrease in the relative value of arrival time of action potential indicates an increase in the propagation speed of the action potential.

FIG. 4 is a diagram showing the results obtained by measuring the effect of picrotoxin by changing its concentration on the propagation speed of action potential in a neural network included in a nerve cell device having a different cell seeding density. As nerve cells, XCL-1 neurons (Xcell Science, US) were seeded at density of 3×10⁵ cells/cm² (A) and 9×10⁵ cells/cm² (B) or cerebral cortex neurons derived from human iPS cells (Axol Bioscience, UK) were seeded at density of 8×10⁵ cells/cm² (C) and 24×10⁵ cells/cm² (D) and cultured for 6 weeks. The concentrations of picrotoxin were 0.1, 0.3, 1, 3 and 10 µM. The results are relative values in which the propagation speed of action potential in case of the addition of vehicle (DMSO, a solvent in the drug) is taken as 100, and indicated by the average ± standard error (A, n=3; B, n=6; C, n=2; D, n=4).

### Embodiments for Carrying out the Invention

The cell scaffold used in the nerve cell device used in the present invention is composed of fibers produced from polymeric materials. The cell scaffold is preferably a fiber sheet in shape of a sheet in which fibers are integrated. The fiber sheet can have an orientational structure, a non-orientational structure, or a mixed structure of orientational and non-orientational structure. The orientational structure is a structure in which fibers constituting the fiber sheet are arranged in one direction. When the angle in the direction is taken as 0°, 80% or more of the fibers are present in the range of ±30°. In the orientational structure, the distance between the fibers (the distance between the core wires of the adjacent fibers) is not particularly limited. However, it is preferably 5-50 µm. The non-orientational structure is a structure in which fibers are randomly arranged in different direction. A polymeric material constituting the fibers is preferably biodegradable or non-biodegradable polymeric material, including but not limited to PLGA (polylactic acid polyglycolic acid), polystyrene (PS), polysulfone (PSU), and polytetrafluoroethylene (PTFE). The diameter of the orthogonal cross section of the fibers constituting the fiber sheet is not particularly limited. However, it is, for example, 0.1-8 µm, preferably 0.5-7 µm, and more preferably 1-6 µm. The thickness of the fiber sheet is, for example, 1-40 µm, preferably 5-35 µm, and more preferably 10-30 µm. The porosity of the fibers constituting the fiber sheet can vary with the polymeric material used. The opening ratio is not particularly limited. However, it is, for example, 10-50%, preferably 15-45%, and more preferably 20-40%. The opening ratio is the ratio of the area where the fibers do not exist with respect to a fixed area of fiber sheet plane.

A fiber sheet can be produced, for example, by electrospinning method from a solution containing a polymeric material. In the case of producing a fiber sheet having an orientational structure, a fiber sheet can be produced for example, by using a rotary drum, spraying a solution containing a polymeric material from a nozzle to a rotating surface of the drum while rotating the drum, and winding the fibers formed on the rotary drum. However, it is not limited to this method. In the case of producing a fiber sheet having a non-orientational structure, a fiber sheet can be produced by spraying a solution containing a polymeric material onto a flat plate. In the case of producing a fiber sheet having a mixed structure of orientational and non-orientational structure, for example, a method for producing a fiber sheet having an orientational structure and that for producing a fiber sheet having a non-orientational structure can be used in combination.

For polytetrafluoroethylene (PTFE) sheet, for example, POREFLON® which is commercially available from Sumitomo Electric Fine Polymer, Inc. can be used.

For a solution of polymeric material, any organic solvent can be used as long as it can dissolve the polymeric material used at a concentration of 10-30 wt% at room temperature. The examples include 1,1,1,3,3,3-hexafluoro-2-propanol (HFIP), N,N-dimethylformamide (DMF), and the like.

A fiber sheet can be fixed or held by a frame around its periphery. When a frame is fixed or held on a fiber sheet, the frame and the fiber sheet can be bonded by using an adhesive, which is not particularly limited unless affecting cell culture. For example, a commercially available biocompatible adhesive, such as silicone one-liquid condensation type RVT rubber (Shin-Etsu Chemical, catalog number: KE-45) can be used.

The material of a frame is not particularly limited unless affecting cell culture. For example, polydimethylsiloxane (PDMS), PS, polycarbonate, stainless steel and the like are exemplified. The thickness of the frame is not particularly limited. However, it is 0.1-4 mm, preferably 0.25-3 mm, and more preferably 0.5-2 mm.

The shape of a frame can be changed according to the purpose of its use. A frame of 2 mm×2 mm to 15 mm×15 mm in terms of length × width is preferable, and in circular or polygonal shape.

A nerve cell device using a fiber sheet or a fiber sheet fixed or held by a frame as cell scaffold can be arranged as it is in at least one of the wells included in a multi-well plate having a cell culture dish or a plurality of wells.

In this specification, nerve cells refer to neural units composed of cell bodies, dendrites and axons, and are also referred to as neurons. Nerve cells can be classified according to the neurotransmitters produced by the nerve cells. As the neurotransmitters, non-peptide neurotransmitters such as monoamine (such as dopamine, noradrenaline, adrenaline and serotonin), acetylcholine, γ-aminobutyric acid and glutamic acid, and peptide neurotransmitters such as an adrenal cortex stimulation hormone (ACTH), α-endorphin, β-endorphin, γ-endorphin and vasopressin can be exemplified. For example, nerve cells containing dopamine, acetylcholine and glutamic acid as transmission substances are referred to as dopaminergic neurons, cholinergic neurons and glutamatergic neurons, respectively.

Primary cultured cells can be used as nerve cells. Since the primary cultured cells retain a large number of cell functions inherent in the living organism, they are important as a system for assessing effects of drugs or the like in the living organism. As primary cultured cells, nerve cells of the central nervous system and peripheral nervous system of mammal, for example, rodent, such as mouse and rat, or primate, such as monkey and human, can be used. When preparing and culturing these nerve cells, methods for anatomy of animals, tissue collection, nerve separation and isolation, a culture medium for nerve cell culture, and culture conditions, etc. can be selected from known methods according to the type of cells to be cultured and the purpose of the cells. As commercially available products of primary cultured cells, for example, rat brain nerve cells of Lonza (Swiss) and human brain nerve cells of ScienCell Research Laboratories (US) can be used.

Moreover, nerve cells derived from pluripotent stem cells can be used as nerve cells. As pluripotent stem cells, for example, there are embryonic stem cells (ES cells) and iPS cells. Various types of nerve cells can be obtained by differentiation induction of pluripotent stem cells by using a known neural differentiation induction method. For example, nerve cells can be obtained by a differentiation induction method using a low-molecular compound described in a literature (Honda et al., Biochemical and Biophysical Research Communication, 469 (2016) 587-592). Besides, commercially available products of nerve cells derived from pluripotent stem cells can also be used. For example, iCell neuron of Cellular Dynamics International (US), which is a product in which iPS cells are differentiated into nerve cells by treating with a predetermined compound, various neural stem cells of Axol Bioscince (UK), the precursor cells of various nerve cells of BrainXell (US), and XCL-1 neurons of Xcell Science (US). Furthermore, SynFire nerve cells of NeuCyte (US) which are products differentiated into nerve cells by introducing a prescribed gene into iPS cells and various nerve cells of Elixirgen Scientific (US) can also be used. These commercially available nerve cells can be cultured by using an attached culture medium.

Nerve cells can be cultured with glial cells differentiated from mammalian brain-derived glial cells or mammalian iPS cells. As glial cells, astrocyte, oligodendrocyte, microglia and the like can be exemplified. In addition, the culture medium (astrocyte culture supernatant) remained after culturing astrocyte can be added to a culture medium for nerve cells at a final concentration of 5-30% and cultured.

A neural network is a neural circuit. A nerve cell having an elongated axon and a plurality of complex-branched dendrites is connected to another nerve cell through these neurites to form the neural circuit as a result. In the neural circuit, when nerve cells (pre-synaptic cells) fire electrically (change in action potential), the electrical signals conducted through axons transmit electrical signals from the pre-synaptic cells to the dendrites of the next nerve cells (post-synaptic cells) by neurotransmitters. A site where an axon and a dendrites are connected is a synapse. In this specification, the neural network is formed by culturing on a cell scaffold a plurality of cells, instead of a single cell, selected from nerve cells derived from stem cells, plant nerve cells, primary nerve cells, stem cells and neural progenitor cells that differentiate into nerve cells in a culture process, as well as cells constituting a neural tissue such as glial cells.

Nerve cells suspended in a culture medium are seeded to a fiber sheet or a fiber sheet fixed or held by a frame around periphery at a density of 1×10⁴ cells/cm² to 4×10⁶ cells/cm², preferably 5×10⁴ cells/cm² to 3×10⁶ cells/cm², more preferably 1×10⁵ cells/cm² to 2×10⁶ cells/cm² and cultured for 7-14 days while changing the culture medium at intervals of 1-7 days. Thus, a nerve cell device in which the nerve cells are uniformly formed into three-dimensional structure can be produced. The formation of a three-dimensional structure refers to a state in which nerve cells grow on one side or both sides of a fiber sheet, or enter into and grow inside a fiber sheet. When nerve cells are seeded on a fiber sheet having an orientational structure, nerve cells are bonded along fibers constituting the fiber sheet and extend along the orientation direction of the fibers

In order to facilitate adhesion and extension of the seeded nerve cells, a fiber sheet may be coated with an extracellular matrix protein, such as polylysine, polyornithine, laminin, fibronectin, Matrigel® and Geltrex®, or polyethyleneimine which is a cationic water-soluble polymer. The coating can be carried out by immersing a fiber sheet in a solution in which the extracellular matrix protein or polyethyleneimine is dissolved in physiological saline, phosphate buffered saline, cell culture medium, or the like.

In order to measure the effect of a drug or the like accurately in the propagation of neural activity in a neural network, it is preferable to measure while securing a constant culture area. From the point of view, it is preferable that the nerve cell device has a size of at least 500 µm×500 µm in terms of length × width. Nerve cells may be seeded on a fiber sheet having this size or may be seeded on a larger fiber sheet and then trimmed to a predetermined size.

For measuring the propagation of electric activity in a neural network included in a nerve cell device and/or an axon, the nerve cell device used in the present invention is capable of contacting a plurality of regularly arranged electrodes and measuring the extracellular potential of the nerve cells contained in the nerve cell device. The measurement of the extracellular potential of nerve cells is normally carried out in an environment of 5% CO₂ and 37°C. The regularly arranged electrodes are electrode array in which a large number of planar microelectrodes electrically insulated from each other are arranged regularly on an electrode substrate, and electric signals from the plurality of cells can be simultaneously observed. The electrodes are, for example, platinum black plating electrodes or carbon nanotube plating electrodes. By regularly arranging the electrodes, the electrodes are kept at a predetermined distance from each other, so that the propagation speed of electric activity in a neural network and/or an axon can be calculated easily and accurately. As such an electrode array, a microelectrode array wherein n×n microelectrodes (where n is an integer, and preferably 4-10000) on a planar substrate, having a size of 10 µm×10 µm to 80 µm×80 µm are arranged in an inter-electrode distance of 100 µm to 500 µm, in an array size of 0.5 mm×0.5 mm to 5 mm×5 mm is preferable.

In addition to the microelectrode array, a fluorescent calcium indicator, such as a calcium-sensitive dye and a calcium-sensitive fluorescent protein, and a fluorescent potential indicator such as a potential-sensitive dye and a potential-sensitive fluorescent protein are used as means for measuring neural activity (Grienberger, C. and Konnerth, A., Neuron, 73, 862-885, 2012; Antic, S. D., et al. J Neurophysiol 116: 135-152, 2016; Miller, E. W., Curr Opin Chem Biol. 33: 74-80, 2016). By using these indicators, variations in calcium and potential of nerve cells included in the nerve cell device of the present invention can also be determined with a cell imaging device.

### Examples

The present invention is described in detail with reference to the following examples. However, the present invention is not limited thereto.

### Example 1

### Production of a fiber device

### (1) Production of a random fiber sheet

PLGA (Sigma, P1941) or PSU (Sigma, 182443) was dissolved in HFIP (Wako Pure Chemicals Corporation, 089-04233) at room temperature to make a concentration of 20 wt%. The solution was filled into a syringe (Norm-Ject Syringes, 5 mL, Osaka Chemical Ind. Co., Ltd.) and then mounted on a nanofiber electric field spinning device NANON-03 (MEC Company Ltd.) to produce a random fiber sheet under the conditions of needle diameter: 22 G, voltage: 20 kV and feed speed: 1 mL/h in the case of PLGA; or under the conditions of needle diameter; 27 G, voltage: 15 kV and feed speed: 1 mL/h in the case of PSU.

### (2) Production of an oriented fiber sheet

To produce an oriented PLGA fiber sheet, PLGA (Sigma, P1941)) was dissolved in HFIP (Wako Pure Chemical Corporation, 089-04233) at room temperature to make a concentration of 20 wt%. The solution was filled into a syringe (Norm-Ject Syringes, 5 mL, Osaka Chemical Ind. Co. Ltd.), then mounted on a nanofiber electric field spinning device NANON-03 (MEC Company Ltd.), to produce the fiber sheet under the conditions of needle diameter on the drum collector: 22 G, voltage: 20 kV, feed speed: 1 mL/h and rotation speed: 750 rpm. To produce an oriented PS fiber sheet, PS (Fluka) was dissolved in DMF (N,N-dimethylformamide, Wako Pure Chemical Corporation) at room temperature to make a concentration of 30 wt%. The solution was filled into a syringe (Norm-Ject Syringes, 5 mL, Osaka Chemical Ind. Co. Ltd.) and then mounted on a nanofiber electric field spinning device NANON-03 (MEC Company Ltd.), to produce the fiber sheet under the conditions of needle diameter on the drum collector: 25 G, voltage: 10 kV, feed speed: 1.5 mL/h and rotation speed: 2000 rpm.

### (3) Frame adhesion to a fiber sheet

A polycarbonate frame (15 mm×15 mm) or a stainless circular frame (outer diameter: 6 mm, inner diameter: 3 mm) was bonded to the fiber sheet produced using a silicone one-liquid condensation-type RVT rubber (Shin-Etsu Chemical, catalog number: KE-45) to produce a fiber device.

### Example 2

### Determination of neural activity propagation by MEA in a neural network constructed on a cell scaffold

A coating solution of SureBond® (Axol Bioscience, UK, catalog number: ax0052) was added to the oriented PLGA fiber sheet produced in Example 1 and coated at 37°C for 1 hour under 5% CO₂ environment. The coated oriented PLGA fiber sheet was used as a cell scaffold. Cerebral cortex neurons derived from human iPS cells (Axol Bioscience, UK, catalog number: ax0019) were seeded on the oriented PLGA fiber sheet at a density of 2.4×10⁶ cells/cm². This was cultured for 5 weeks in an incubator under the conditions of 5% CO₂ and 37°C using BrainPhys culture (STEMCELL Technologies, US) containing 100 U/mL penicillin/streptomycin (Wako Pure Chemical Corporation). After the culturing, the cell sheet obtained was placed on a microelectrode array (MEA) probe (MED64 system, Alpha MED Scientific Inc.) to bring the cells into contact with the electrode of the MEA probe, and measure neural activity (spike firing) and the propagation direction of neural activity. The results are shown in FIG. 1.

The orientation direction of the fiber constituting the fiber sheet against the microelectrodes arranged in the MEA was the direction of the diagonal line connecting the channel 1 and the channel 64. The nerve cells extended along the orientation direction (FIG. 1A). The integrated values of the spike number of all electrodes (AWSDR) increased with time, and then decreases (FIG. 1B). The lapse of time of the spike measured at each electrode was found to delay from the channel 64 toward the channel 1 (indicated by an arrow dotted line in FIG. 1C). This indicates that the orientation direction of the fibers, that is, the propagation of neural activity along the extension direction of the nerve cells was observed. Accordingly, it is indicated that by using the method of using a nerve cell device according to the present invention, neural activity can be assessed by using the propagation speed of neural activity as an index.

### Example 3

### The effect of tetrodotoxin (TTX) on the propagation speed in axon of the action potential in single neuron

The oriented PLGA fiber sheet produced in Example 1 was coated in the same manner as in Example 2. This oriented fiber sheet was used as a cell scaffold. A spinal motion neuron derived from human iPS cells (BrainXell, US) was suspended in a Seeding Medium (BrainXell) containing BDNF (brain derived neurotrophic factor), GDNF (glial-cell derived neurotrophic factor) and TGF (transforming growth factor)-β1, and seeded on the oriented PLGA fiber sheet at a density of 1.4×10⁶ cells/cm². The cells were cultured for 6 weeks according to the cell culture procedure attached to the cell culture kit of BrainXell. The resulting cell sheet was placed on a microelectrode array (MEA) probe (MED64 system, Alpha MED Scientific Inc.) to bring the cells into contact with the electrode of the MEA probe. This was used for the determination of the action potential. Tetrodotoxin (TTX), a blocker of voltage-gated sodium channel, was added, and its effect on the propagation speed in axon of action potential was examined. FIG. 2A shows the action potential measured over time for channels 2, 10, 18 and 26. Compared to that before the addition of TTX (Before), a phenomenon in which the peak time of action potential delayed due to addition of TTX (100 nM) was confirmed. Regardless of the presence or absence of TTX, a spike was acquired by a plurality of electrodes within 1 msec. Thus, it is indicated that the neural activity of single neuron has been observed. FIG. 2B is a diagram comparing the propagation speed in axon of the action potential before the addition of TTX (Before) and after the addition of TTX (100 nm). (Each result is indicated as average value ± standard error.) The propagation speed in axon of the action potential was 129±14 µsec (n=12) before the addition of TTX. However, it decreased significantly to 170±13 µsec (n=9) after the administration of TTX (p<0.05, corresponding two-side t test). The results show that by using the method of the present invention, quantitative drug assessment regarding propagation speed of action potential in axon is possible.

### Example 4

### The effect of a convulsive drug (4-aminopyridin (4-AP)) on neural activity propagation in neural network of nerve cell device

An oriented PS fiber sheet obtained by mounting a stainless steel circular frame produced in Example 1 was hydrophilized by plasma treatment using a desktop plasma treatment apparatus (STREX Inc.), and then coated with poly-D-lysine and laminin. Cerebral cortex neurons derived from human iPS cells (Axol Bioscience, UK) was seeded on the coated oriented PS fiber sheet at a density of 8×10⁵ cells/cm² and cultured in an incubator at 5% CO₂ and 37°C for 6 weeks to produce a nerve cell device. The resulting nerve cell device was placed on a probe of a microelectrode array (MEA) MED64-Presto (Alpha MED Scientific Inc.) to bring the cell into contact with the electrode of the MEA probe. The nerve action potential and the propagation direction of the neural activity were measured.

The propagation direction of neural activity in the neural network included in the nerve cell device is the direction from the channel number 1 to the channel number 16 of the MEA, (Each channel is at the diagonal of the MEA.) and is indicated by an arrow in FIG. 3A (raster plot drawing). In case of addition of DMSO, a solvent, the propagation speed of neural activity was 0.14 m/sec (FIG. 3, upper figure). In case of addition of 4-AP (3 µM), a drug which excites nerve cells by blocking potassium channel on the cell membrane, the propagation speed of neural activity increased to 0.39 m/sec (FIG. 3A, lower figure). Since the decrease in relative values of action potential arrival time indicates an increase in propagation speed of action potential, the propagation speed of neural activity was shown to increase dependently with the concentration of 4-AP (FIG. 3B). It is shown by these results that by using the method of the present invention, drug effect on nerve cell function or drug toxicity to nerve cells can be assessed using the propagation speed of neural activity as an index.

### Example 5

### The effect of a convulsive drug (picrotoxin) on neural activity propagation in neural network included in a nerve cell device having different cell seeding density

An oriented PS fiber sheet obtained by mounting a stainless steel circular frame produced in Example 1 was hydrophilized by plasma treatment using a desktop plasma treatment apparatus (STREX Inc.), and then coated with poly-D-lysine and laminin. Nerve cells derived from human iPS cells (XCL-1 neuron, XCell Science, US) were seeded at a density of 3×10⁵ cells/cm² or 9×10⁵ cells/cm², or cerebral cortex neurons derived from human iPS cells (Axol Bioscience, UK) were seeded at a density of 8×10⁵ cells/cm² or 24×10⁵ cells/cm², respectively on the coated oriented PS fiber sheet and cultured in an incubator at 5% CO₂ and 37°C for 6 weeks. Each of the resulting cell sheets was placed on a probe of a microelectrode array (MEA) MED64-Presto (Alpha MED Scientific Inc.) to bring the cells into contact with the electrode of the MEA probe. The nerve action potential was determined.

Picrotoxin is a drug that excites nerve cells by blocking GABA_{A} receptor which is one of the receptors of γ-aminobutyric acid (GABA). Even when any cells of XCL-1 neuron or cerebral cortex neuron derived from human iPS cells were used as nerve cells constituting a nerve cell device, by the addition of picrotoxin, an increase in propagation speed of a concentration-dependent action potential was observed in a picrotoxin concentration range of 0.1-10 µm (FIGS. 4A-D). In addition, even when the cell seeding density onto the fiber sheet is different, an increase in the propagation speed of the action potential is observed in a picrotoxin concentration-dependent manner (FIGS. 4A and B or FIGS. 4C and D). It is shown by these results that by using the method of the present invention, drug effect on nerve cell function or drug toxicity to nerve cells can be assessed using the propagation speed of neural activity as an index. Besides, the method of the present invention has been shown to be applicable to a plurality of nerve cells. Further, it has been shown that in the production of a nerve cell device used in the present invention, even when cell seeding density is changed, drug assessment using the propagation speed of neural activity as an index is possible.

## Claims

1. A method of measuring propagation of electric activity in a neural network and/or axon using a nerve cell device which comprises a cell scaffold and nerve cells.

2. The method according to claim 1, wherein the cell scaffold is a fiber sheet formed of a polymeric material.

3. The method according to claim 2, wherein the fiber sheet has an orientational structure, a non-orientational structure, or a mixed structure of orientational and non-orientational structure.

4. The method according to claim 2 or 3, wherein the fiber sheet is coated with an extracellular matrix protein selected from polylysine, polyornithine, laminin, fibronectin, Matrigel® and Geltrex®.

5. The method according to claim 2 or 3, wherein the fiber sheet is coated with polyethyleneimine.

6. The method according to any one of claims 1 to 5, wherein the nerve cells have formed a three-dimensional structure on a cell scaffold and/or in a cell scaffold.

7. The method according to any one of claims 1 to 6, wherein the nerve cells are the nerve cells derived from primary cultured cells or pluripotent stem cells.

8. The method according to claim 7, wherein the nerve cells derived from primary cultured cells or pluripotent stem cells are those derived from mammals.

9. The method according to any one of claims 1 to 8, wherein the nerve cells comprise glutamatergic, dopaminergic, γ-aminobutyric acidergic, monoaminergic, histaminergic or cholinergic nerve cells.

10. The method according to any one of claims 1 to 9, wherein the nerve cell device further comprises a frame that holds the periphery of the device.

11. The method according to claim 10, wherein the frame has a size of 2 mm×2 mm to 15 mm×15 mm in terms of length × width, and has circular or polygonal shape.

12. The method according to any one of claims 1 to 11, wherein the nerve cell device is in contact with a plurality of regularly arranged electrodes.

13. The method according to claim 12, wherein the regularly arranged electrodes are microelectrode arrays.

14. A method for assessing neural activity using the method according to any one of claims 1 to 13.
